# EUROPÄISCHE PATENTSCHRIFT

(11) **EP 2 352 814 B1**
(45) Veröffentlichungstag und Bekanntmachung des Hinweises auf die Patenterteilung: **08.04.2015**
(21) Anmeldenummer: 09756745.7
(22) Anmeldetag: 24.11.2009
(51) Int. Cl.: C12N 1/14, C12P 7/26

(54) **ENZYMATISCHE SYNTHESE VON NOOTKATON**
ENZYMATIC SYNTHESIS OF NOOTKATONE
SYNTHÈSE ENZYMATIQUE DE NOOTKATONE

(30) Priorität: 25.11.2008 EP 08169898; 10.12.2008 EP 08171148
(43) Veröffentlichungstag der Anmeldung: 10.08.2011
(73) Patentinhaber: N-Zyme BioTec GmbH, 64295 Darmstadt (DE)
(72) Erfinder: ZORN, Holger, 35435 Wettenberg (DE); FRAATZ, Marco, Alexander, 35392 Giessen (DE); RIEMER, Stephanie Johanna Luise, 30659 Hannover (DE); TAKENBERG, Meike, 30826 Garbsen (DE); KRINGS, Ulrich, 31628 Landesbergen (DE); BERGER, Ralf Günter, 30455 Hannover (DE); MARX, Stefan, 64380 Roßdorf (DE)
(74) Vertreter: Fabry, Bernd
(86) Internationale Anmeldenummer: PCT/EP2009/065716
(87) Internationale Veröffentlichungsnummer: WO 2010/060898

(56) Entgegenhaltungen:
- WO-A-2005/078110
- FURUSAWA MAI ET AL: "Highly efficient production of nootkatone, the grapefruit aroma from valencene, by biotransformation." CHEMICAL & PHARMACEUTICAL BULLETIN NOV 2005, Bd. 53, Nr. 11, November 2005 (2005-11), Seiten 1513-1514, XP002518325 ISSN: 0009-2363 in der Anmeldung erwähnt
- DATABASE UniProt [Online] 21. August 2007 (2007-08-21), "SubName: Full=Putative uncharacterized protein;" XP002518326 gefunden im EBI accession no. UNIPROT:A6SG40 Database accession no. A6SG40
- DATABASE EMBL [Online] 10. Januar 2009 (2009-01-10), "Pleurotus sapidus mRNA for valencene oxygenase (aOx1 gene)" XP002518491 gefunden im EBI accession no. EMBL:FM200795 Database accession no. FM200795

## Beschreibung

Die vorliegende Erfindung betrifft Verfahren zur Oxidation von Terpenkohlenwasserstoffen und dafür einsetzbare Polypeptide.

Während Terpenkohlenwasserstoffe häufig als unerwünschte Überschusskomponenten aus etherischen Ölen entfernt werden, finden synthetische oxyfunktionalisierte Derivate breite Verwendung als Aroma- und Duftstoffe. Dieses Missverhältnis im Verein mit den Spurenvorkommen der wirtschaftlich interessanten Terpenoide in pflanzlichen Quellen hat der mikrobiellen Terpenbiotransformation eine inzwischen fünfzigjährige Geschichte beschert (Schrader und Berger 2001). (+)-Nootkaton besitzt einen an Citrus und Grapefruit erinnernden Geruch, einen leicht bitteren Geschmack und einen extrem niedrigen sensorischen Schwellenwert von etwa einem µg L⁻¹ Wasser (Ohloff 1994). Diese Eigenschaftskombination hat Nootkaton weltweit zu einem vielgesuchten Bioprodukt werden lassen.

Die erste in der Literatur erwähnte Biotransformation von Valencen stammt aus dem Jahr 1973 (Dhavlikar und Albroscheit 1973). Valencen wurde mit zwei isolierten *Enterobacter*-Arten mit einer maximalen Ausbeute von 12% (w/w) zu (+)-Nootkaton transformiert. Die Biotransformation von Valencen mit Zellkulturen von Grapefruit (*Citrus paradisi*) führte nach sechs Stunden Inkubationsdauer zu Nootkatongehalten von *ca.* 1 mg L⁻¹ (Drawert *et al.* 1984). Del Rio *et al.* verwendeten verschiedene *Citrus*-Arten zur Biosynthese von Nootkaton und erzielten die höchsten Nootkatonausbeuten mit neun Monate alten Kalluskulturen von *Citrus paradisi* (1,6 µg g⁻¹ Biofeuchtmasse). 1994 wurde die Biosynthese von Nootkaton durch einen *Rhodococcus*-Stamm (KSM-5706) beschrieben (Okuda *et al.* 1994), jedoch waren die Ausbeuten ebenfalls gering.

Unter den neuartigen Lösungsansätzen der jüngsten Zeit sind Experimente mit rekombinanten Mikroorganismen und mit Pflanzenzellkulturen beziehungsweise pflanzlichen Präparationen zu nennen. Mikrosomale Präparationen aus der Wurzel von Chicoree (*Cichorium intybus* L.) zeichneten sich durch eine vernachlässigbare Bildung von Nebenprodukten aus (Bouwmeester *et al.* 2007; de Kraker *et al.* 2003), jedoch führt auf absehbare Zeit kein gangbarer Weg zu ausreichenden Mengen dieses Biokatalysators. Die Oxyfunktionalisierung von (+)-Valencen mit rekombinanten P450_{cam}-Enzymen aus *Pseudomonas putida* mit einer maximalen Ausbeute von 9% (w/w) wurde 2005 publiziert (Sowden *et al.* 2005). Die Umsetzung mit rekombinanten P450_{BM-3}-Enzymen wurde ebenfalls beschrieben, führte neben (+)-Nootkaton jedoch zu einer Reihe weiterer Produkte.

Auch Submerskulturen des Ascomyceten *Chaetomium globosum* wurden zur Darstellung von (+)-Nootkaton aus (+)-Valencen eingesetzt (Kaspera *et al.* 2005). Nach dreitägiger Transformation wurden 8 mg L⁻¹ (+)-Nootkaton erzielt, wobei wiederum zahlreiche flüchtige und nicht flüchtige Nebenprodukte gebildet wurden. Außerdem sind Pflanzenzellkulturen von *Gynostemma pentaphyllum, Caragana chamlagu* und *Hibiscus cannabinus* zur Nootkatonsynthese aus (+)-Valencen befähigt (Sakamaki *et al.* 2005). Maximale Ausbeuten an (+)-Nootkaton wurden mit *Gynostemma pentaphyllum* (Cucurbitaceae) jedoch erst nach 20-tägiger Transformation erzielt (600 mg L⁻¹). Lange Inkubationszeiten waren auch bei Submerskulturen von *Mucor* sp. und *Chlorella pyrenoidosa* erforderlich (Hashimoto *et al.* 2003b, Hashimoto *et al.* 2003a; Furusawa *et al.* 2005).

Zur Umgehung der mit der Verwendung von intakten Zellen assoziierten Probleme ist der Einsatz isolierter Enzyme vorgeschlagen worden. Ein Schweizer Aromaproduzent beschreibt die Zugabe von Lipoxygenase und ungesättigten Fettsäuren zu Valencen (Muller *et al.* 1998). Laccasen benötigten synthetische Cosubstrate (Hitchman *et al.* 2005) oder einen zweiten Reaktionsschritt (Erhitzen/Schwermetallkatalyse) (Huang *et al.* 2001). Mit Lyophilisaten aus Basidiomycten wurden gute Raum-Zeitausbeuten erzielt, ohne dass die einschlägige Patentanmeldung ein konkretes Verfahrensbeispiel beschreibt (Müller *et al.* 2005).

Es besteht daher weiterhin ein Bedarf nach Verfahren, die es erlauben, Terpenkohlenwasserstoffe mit hoher Effizienz umzusetzen.

Aufgabe der vorliegenden Erfindung war es, ein solches Verfahren bereitzustellen.

Gelöst wird die Aufgabe durch die Bereitstellung eines Polypeptides, das zur Oxidation von Terpenkohlenwasserstoffen geeignet ist, sowie der nötigen Angaben, um das Enzym rekombinant herstellen zu können.

Gegenstand ist daher zum einen ein Polypeptid mit einer Aminosäuresequenz gemäß SEQ ID No. 1 oder eine Variante, bei der bis zu 10% der Aminosäuren durch Insertionen, Deletionen oder Substitution verändert sind, die Terpenkohlenwasserstoffe oxidieren kann.

Insertion bedeutet die Einfügung genau einer Aminosäure; Deletion die Entfernung genau einer Aminosäure. Bei einer Substitution wird eine Aminosäure durch eine andere Aminosäure ersetzt.

Aminosäuren sind neben den 20 natürlich vorkommenden Aminosäuren auch Aminosäurederivate, wie z.B. Hydroxyprolin, Derivate, die durch Veresterung von Carbonsäuren oder durch Amidbildung oder ähnliches erhalten werden können. Neben den natürlichen L-Aminosäuren können auch D-Aminosäuren zum Einsatz kommen.

Bevorzugt kommt ein Peptid zum Einsatz, bei dem weniger als 10% der Aminosäuren durch Insertionen, Deletionen oder Substitution verändert sind, insbesondere maximal 8%, maximal 5%, maximal 3% und besonders bevorzugt maximal 1%.

In einer Ausführungsform weisen die Peptide C-terminale und/oder N-terminale Trunkierungen auf.

Eine bevorzugte Ausführungsform des Proteins ist ein Protein, dass durch SEQ. ID. No. 2 oder eine Nukleinsäure, die unter stringenten Bedingungen mit dieser hybridisiert, kodiert wird.

Das erfindungsgemäße Peptid zeigt die Eigenschaften eines Enzym, nämlich einer Oxygenase.

Das Enzym ist biochemisch und molekularbiologisch charakterisiert und zeigt nur geringe Sequenzhomologien zu bislang bekannten Oxygenasen, sei es aus Pilzen oder aus anderen Mikroorganismen.

Gegenstand der Erfindung ist weiterhin eine Nukleinsäure, die für das erfindungsgemäße Polypeptid kodiert und eine besonders bevorzugte Ausführungsform hiervon ist die Sequenz gemäß Seq. ID Nr. 2. oder mit dieser unter stingenten Bedingungen hybridisieren.

Ein weiterer Gegenstand der Erfindung ist ein Vektor, der die erfindungsgemäße Nukleinsäure enthält, sowie ein transformierter Organismus, der den erfindungsgemäßen Vektor enthält.

Gegenstand des Verfahrens ist weiterhin ein Verfahren zur Oxidation von Terpenkohlenwasserstoffen umfassend den Schritt des
- Inkontaktbringens von Terpenkohlenwasserstoffen mit einem Polypeptid gemäß Anspruch 1.

Eingesetzt werden kann also ein aufgereinigtes Enzym aus natürlichen Quellen oder rekombinanter Herstellung. Ein aufgereinigter Enzym kann aus dem Mycel eines Basidiomyceten erhalten werden, nachdem dieses physikalisch, chemisch oder enzymatisch aufgeschlossen wurde.

Bevorzugt erfolgt ein solcher Aufschluss durch Dispergieren, Kugelmahlen oder Hochdruckhomogenisieren.

Eine Gefriertrocknung oder Lyophilisierung ist kein Aufschluss; das alleinige Lyophilisieren führt daher nicht zum gewünschten Ergebnis. Ausgeschlossen ist daher die alleinige Lyophilisierung ohne weitere Homogenisierungsschritte.

Als Basidiomyceten eignen sich insbesondere Basidiomyceten aus der Familie der Pleurotaceae, insbesondere *Pleurotus sapidus.*

Besonders bevorzugt ist das erfindungsgemäße Verfahren einsetzbar, um aus Valencen Nootkaton zu erhalten. Es sind aber auch beliebige weitere Terpenkohlenwasserstoffe einsetzbar.

Die Erfindung wird durch die nachfolgenden Beispiele näher erläutert.
Figur 1 zeigt die Produktausbeuten nach Transformation von (+)-Valencen mit aufgeschlossener Biofeuchtmasse; Transformation von 2 µL (+)-Valencen mit Biofeuchtmasse (BFM, aufgeschl.) in 1,5 mL 50 mM TRIS-HCl, pH 7,5 für 16 Stunden.
Figur 2 zeigt die Produktausbeuten nach Fed-Batch Transformation von (+)-Valencen mit aufgeschlossener Biofeuchtmasse in 100 mL 50 mM TRIS-HCl, pH 7,5 für 50 Stunden.
Figur 3 zeigt ein FPLC-Chromatogrammausschnitt der dritten Stufe der 3-Stufenreinigung von separiertem Überstand an einer Superdex-200-Säule;
Laufpuffer: 200 mM TRIS-HCl, pH 7,5, Flussrate: 0,5 mL min⁻¹, Probenvolumen: 200 µL, Fraktionsgröße: 1,0 mL.
Figur 4 zeigt die Produktausbeuten nach Transformation mit FPLC-Fraktionen der 3. Reinigungsstufe; Transformation von 1 µL (+)-Valencen in einem Volumen von 1,5 mL für 20 Stunden; Puffer: 20 mM TRIS-HCl, pH 7,5.
Figur 5 zeigt ein Gel (12%ig) der SDS-PAGE nach 3-stufiger Proteinreinigung mittels FPLC mit anschließender Silberfärbung; MW = Molekulargewicht, Std. = Standard (1 µL).
Figur 6A zeigt ein Gel Gel (12%ig) der SDS-PAGE nach 3-stufiger Proteinreinigung mittels FPLC mit spezifischer Färbung für Häm-/Metallenzyme (3,3',5,5'-Tetramethylbenzidin); MW = Molekulargewicht des Standards, Std. = Standard (4 µL), MPO = Meerrettich-Peroxidase (15 µL, 345 mU), Über. = separierter Überstand (15 µL), GF = vereinte aktive Fraktionen aus der 3. Reinigungsstufe (Gelfiltration, 15 µL), = entsprechende Probe vor SDS-PAGE 5 min auf 95°C erhitzt.
Figur 6B zeigt ein Gel (12%ig) der SDS-PAGE mit anschließender Färbung für Häm-/Metallenzyme; SLOX = Lipoxygenase aus Sojabohnen (20 µL, 85 mU).
Figur 7 zeigt Alignment der Peptidsequenzen 66-1 und 66-3 aus P. sapidus mit der Sequenz einer Lipoxygenase aus A. fumigatus (XP_746844.1) mittels ClustalW (Thompson et al. 1994).
Figur 8 zeigt die cDNA- und Aminosäuresequenz des erfindungsgemäßen Polypeptides aus P. sapidus, Start- und Stopcodon sind fett markiert.

### Beispiele

### Beispiel 1: Mikroorganismus und Kultivierung

*Pleurotus sapidus* (DSMZ 8266) wurde von der Deutschen Sammlung für Mikroorganismen und Zellkulturen (DSMZ), Braunschweig bezogen.

### a) Nahrlösungen

Die einzelnen Bestandteile (Tabelle 1) wurden in destilliertem Wasser gelöst und mit 0,5 M Natronlauge auf pH 6,0 eingestellt.

**Tabelle 1: Zusammensetzung der Nährlösungen; Glc = D-(+)-Glucose-Monohydrat, Asn = L-Asparagin-Monohydrat, SE-Lsg = Spurenelementlösung (Tabelle 2) = Kon-zentration, * = modifiziert nach Sprecher 1959**

| | **Glc** | **Melasse** | **Asn** | **NH₄NO₃** | **KH₂PO₄** | **MgSO₄** | **HefeExtrakt** | **SE-Lsg** |
|---|---|---|---|---|---|---|---|---|
| | **c [g L⁻¹]** | **c[g L⁻¹]** | **c [g L⁻¹]** | **c [g L⁻¹]** | **c [g L⁻¹]** | **c [g L⁻¹]** | **c [g L⁻¹]** | **c [g L⁻¹]** |
| SNL-H* | 30 | 0 | 4,5 | 0,0 | 1,5 | 0,5 | 3,0 | 1,0 |
| NLMA | 0 | 81 | 0 | 2,4 | 1,5 | 0,5 | 3,0 | 1,0 |

**Tabelle 2: Zusammensetzung der Spurenelementlösung**

| **Substanz** | **Konzentration [g L⁻¹]** |
|---|---|
| FeCl₃ · 6 H₂O | 0,080 |
| ZnSO₄ · 7 H₂O | 0,090 |
| MnSO₄ · H₂O | 0,030 |
| CuSO₄ · 5 H₂O | 0,005 |
| EDTA | 0,400 |

### b) Stammkultivierung

Von *Pleurotus sapidus* wurden Stammkulturen auf Agarplatten mit SNL-H-Agar-Medium angelegt. Dazu wurde je eine Agarplatte mit einem ca. 1 cm² großen, mit Myzel bewachsenen Agarstück beimpft, mit Parafilm^{®} verschlossen und im Brutschrank bei 24°C kultiviert (Taubert *et al.* 2000). Nachdem die Platten zur Hälfte mit Myzel bewachsen waren, wurde die Kultur bei 4°C gelagert. Diese Stammkulturen wurden mindestens alle 6 Monate nach dem gleichen Verfahren überimpft.

### c) Vorkulturen

Ein myzelbewachsenes Agarstück (ca. 1 cm²) der Stammkultur wurde mit Hilfe eines sterilen Spatels in einen Erlenmeyerkolben (500 mL) mit 200 mL SNL-H-Medium überführt, homogenisiert (Ultra-Turrax-Homogenisator, TP 18/10, IKA, Staufen, ca. 20 s bei niedriger Drehgeschwindigkeit) und 4 Tage bei 24°C und 150 U min⁻¹ inkubiert.

### d) Anzucht von Biomasse

Im Rührkesselreaktor wurden 2,3 L NLMA-Medium mit 230 mL homogenisierter Vorkultur inokuliert. Nach 4 Tagen wurde der Rührkesselreaktorinhalt geerntet. Dazu wurde dieser durch ein Baumwolltuch filtriert. Das erhaltene Pilzmyzel wurde zweimal mit je 400 mL destilliertem Wasser (m/v) gewaschen und für weitere Experimente verwendet. Die Anzucht erfolgte unter einer Sterilwerkbank und die eingesetzten Geräte und Lösungen wurden zuvor 20 min bei 121°C autoklaviert.

### Beispiel 2: Zellaufschluss

### a) Homogenisierung von Biofeuchtmasse

3 g Biofeuchtmasse wurden mit 7 mL 50 mM TRIS-HCl, pH 7,5 versetzt und anschließend bei 15.600 U min⁻¹ für 5 min auf Eis mit einem Ultra-Turrax-Homogenisator (TP 18/10, IKA) behandelt. Anschließend wurde das aufgeschlossene Pilzmyzel auf 16,7% (v/v) mit dem selben Puffer verdünnt und zur Transformation von (+)-Valencen verwendet.

### b) Homogenisierung von Biofeuchtmasse- Upscaling

50 g Biofeuchtmasse wurden mit 50 mL 50 mM TRIS-HCl, pH 7,5 versetzt und anschließend bei 20.000 U min⁻¹ für 15 min auf Eis mit einem Ultra-Turrax-Homogenisator (TP 18/10, IKA) behandelt. Anschließend wurde das aufgeschlossene Pilzmyzel auf 25% (v/v) mit dem selben Puffer verdünnt und zur Transformation von (+)-Valencen verwendet.

### c) Homogenisierung von Biofeuchtmasse mit Hochdruck

Pilzmyzel-Suspensionen in VE-Wasser wurden mittels Hochdruck-Homogenisation (LAB 60/60 TBS, Gaulin APV, Schweiz) aufgeschlossen. Gekühltes Mycel wurde mit 1 bis 3 Passagen (150/30 bar und 300/60 bar) aufgeschlossen und direkt für die Biotransformationsreaktion eingesetzt. Alternativ wurde Valencen direkt während des Zellaufschlusses einhomogenisiert.

### d) Herstellung von Lyophilisaten

Bis zu 100 g Biofeuchtmasse wurde in eine Glaspetrischale eingewogen, mit Aluminiumfolie abgedeckt und bei -20°C tiefgefroren. Die Lyophilisierung erfolgte für 3 bis 7 Tage (VaCo 2, Zirbus Technology, Bad Grund). Die Stellflächentemperatur betrug -20°C, die Temperatur der Kühlspirale -45°C. Das erhaltene Lyophillisat wurde ausgewogen, mit einem Glasstab zerkleinert, in sterile Falcon^{™}-Tubes überführt und bis zur Verwendung bei -70°C gelagert.

### Beispiel 3: Biotransformation von Valencen

### a) Transformation mit aufgeschlossener Biomasse

### Die Transformation wurde in Schraubdeckelgläschen (4 mL) in horizontaler

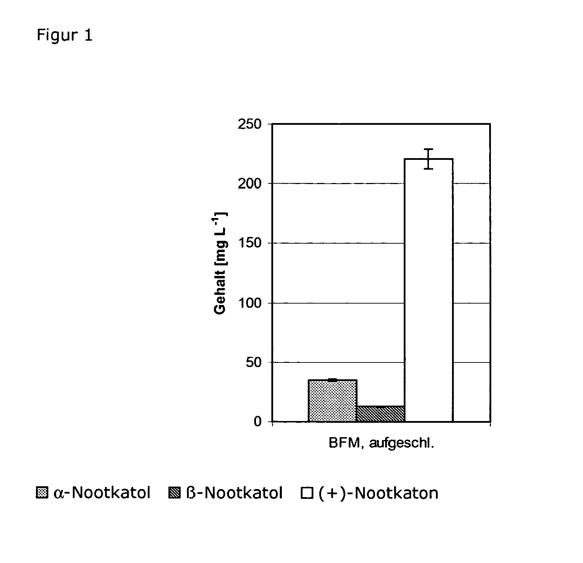

Lage bei 300 U min⁻¹ und 24°C durchgeführt. Biofeuchtmasse von *Pleurotus sapidus (1,5 mL)* wurde mit einem Ultra-Turrax-Homogenisator behandelt und zur Transformation von 2 µL (+)-Valencen (Döhler, 70%) verwendet. Nach dem Ende der Transformation wurde extrahiert. Der ermittelte Gehalt an (+)-Nootkaton betrug 221 mg L⁻¹ (Figur 1).

### b) Fed-Batch Transformation von Valencen

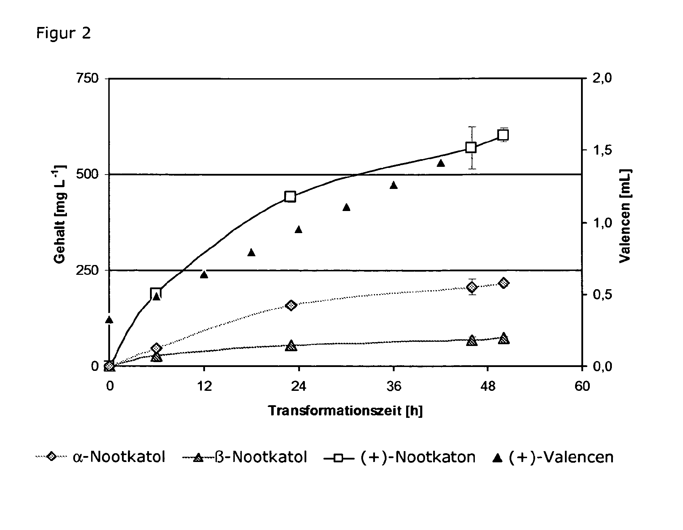

Die Transformation wurde in Glasflaschen (500 mL) auf einem Magnetrührer (Variomag Poly 15, Thermo Fisher Scientific, Waltham, MA, USA) bei 900 U min⁻¹ und Raumtemperatur durchgeführt. Die Transformation wurde durch Zugabe von 333 µL (+)-Valencen (Döhler, ≥ 70%) zu 100 mL Zellsuspension (homogenisiert) gestartet. Nach 6, 12, 18, 24, 30, 36 und 42 h wurde jeweils 156 µL (+)-Valencen zudosiert. Nach 6, 23, 46 und 50 h wurden jeweils 2x 1,5 mL Probe entnommen und extrahiert. Nach 50 h Transformationsdauer betrug der Gehalt an (+)-Nootkaton 603 mg L⁻¹ (Figur 2). In Summe wurden 893 mg L⁻¹ α-, β-Nootkatol und (+)-Nootkaton gebildet.

### c) Transformation mit gereinigter Enzymlösung

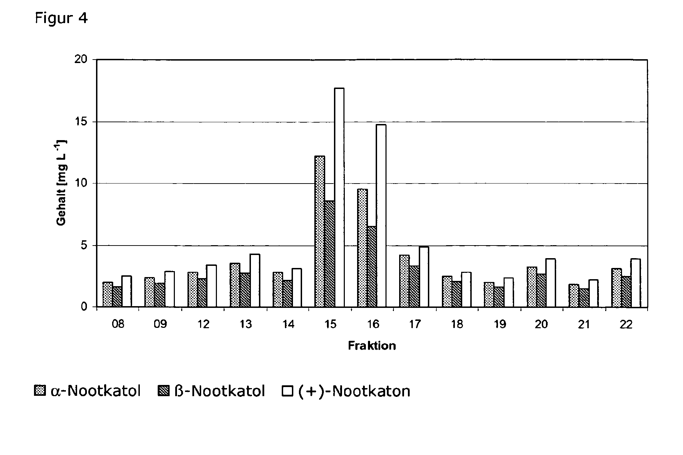

0,5 mL gereinigter Proteinfraktion (Fraktionen 08, 09 und 12 bis 22) wurden mit 1,0 mL 20 mM TRIS-HCl, pH 7,5 gemischt. Anschließend wurde die Transformation durch Zugabe von 1 µL (+)-Valencen (Fluka, ≥ 90%) gestartet. Die Transformation wurde in Schraubdeckelgläschen (4 mL) in horizontaler Lage bei 150 U min⁻¹ und 24°C durchgeführt. Nach dem Ende der Transformation wurde extrahiert. Signifikante Gehalte an den Transformationsprodukten α-, β-Nootkatol und (+)-Nootkaton wurden nach Umsetzung mit den Fraktionen 15 und 16 nachgewiesen (Figur 4).

### d) Kapillargaschromatographie (GC) - Mikroextraktion nach Transformation

Die Mikroextraktion erfolgte direkt in dem für die Transformation verwendeten Schraubdeckelgläschen. Der Ansatz wurde mit internem Standard (67 mg L⁻¹ bzw. 200 mg L⁻¹ Thymol) und 2 mL Pentan versetzt, 10 s gevortext, 10 min bei 150 U min⁻¹ horizontal geschüttelt. Nach Zentrifugation (10 min, 3.313 x g, 4°C) wurde die organische Phase über Natriumsulfat getrocknet und gaschromatographisch untersucht.

### e) Kapillargaschromatographie (HRGC)

Die Quantifizierung von α-, ß-Nootkatol und (+)-Nootkaton mittels FID erfolgte mit einem Kaltaufgabesystem und polarer Trennsäule.

**Tabelle 3: GC-FID (KAS) mit polarer Trennsäule**

| Gaschromatograph | Hewlett Packard HP 6890 Series GC Systems |
|---|---|
| Injektor | Gerstel KAS 4 + Gerstel Controller 505 |
| KAS-Parameter | splitless (0,5 min); 60°C (0 min), 12°C s⁻¹ - 240°C (3 min) |
| KAS-Liner | Gerstel Glasverdampferrohr mit Verwirbelungseinstichen |
| Trennsäule | Varian WCOT Fused Silica CP Wax 52CB, 30 m x 0,25 mm ID, 0,25 µm Filmdicke |
| Detektor | FID, 250°C Gase: H₂: 40 mL min⁻¹, Luft: 450 mL min⁻¹, N₂: 45 mL min⁻¹ |
| Träqerqas | H₂, Vordruck: 58,4 kPa, Fluss: 1,5 ml min⁻¹ (konstant) |
| Datenaufnahme | Hewlett Packard HP GC ChemStation, Version Rev. A.0504 [273] |
| Temperaturprogramm | 40°C (2 min), 5°C min⁻¹ - 150°C (0 min), 3°C min⁻¹ - 222°C (0 min), 10°C min⁻¹ - 240°C (10 min) |
| Autosampler | Hewlett Packard HP 6890 Series Injector (Enhanced Parameters) |

### Beispiel 4: Enzymreinigung

### Fast Protein Liquid Chromatography (FPLC)

Zur Abtrennung von Verunreinigungen wurde die Probe zentrifugiert (10 min, 13.000 U min⁻¹, 16.060 x g, 4 C) und der Überstand in die FPLC (Tabelle 4) injiziert. Wenn nötig, wurde die Probe zusätzlich membranfiltriert (0,45 µm Porengröße, 25 mm, PET, Carl Roth GmbH).

### a) Dreistufenreinigung mittels FPLC

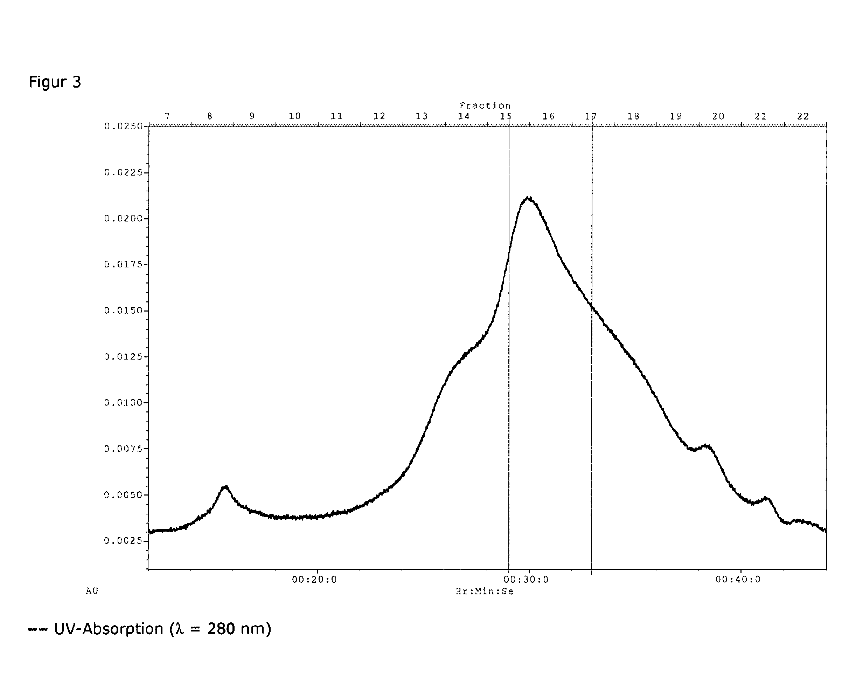

Viermal 5 mL separierter Überstand von *Pleurotus sapidus* wurde jeweils in die FPLC injiziert und mit Hilfe des schwachen Anionenaustauschers DEAE FF getrennt,(siehe Tabelle 5). Die Fraktionen 09 bis 24 der vier FPLC-Läufe wurden vereint (Reinigungsstufe 1, *DEAE-Pool*), mit 20 mM Na-Citrat, pH 3,0 umgepuffert (Centricon Plus-70, Ausschlussgröße 10 kDa) und auf 2,5 mL konzentriert. Ausgefallene Proteine wurden durch Zentrifugation entfernt. 2,0 mL des umgepufferten *DEAE-Pools* wurden erneut in die FPLC injiziert und mittels des starken Kationenaustauschers SP Sepharose FF getrennt, Tabelle 6). Die Fraktionen 07 bis 11 wurden vereint (Reinigungsstufe 2, *SP-FF-Pool*), mit 200 mM TRIS-HCl, pH 7,5 umgepuffert (Amicon Ultra-15, Ausschlussgröße 10 kDa) und auf 300 µL konzentriert. 200 µL des umgepufferten *SP-FF-Pools* wurden in die FPLC injiziert und durch Gelfiltration an einer Superdex-200-Säule (Tabelle 7) getrennt (Reinigungsstufe 3). Nach Kalibrierung der verwendeten Trennsäule entsprach das Peakmaximum (30 min, Figur 3) einem Molekulargewicht von 54 kDa.

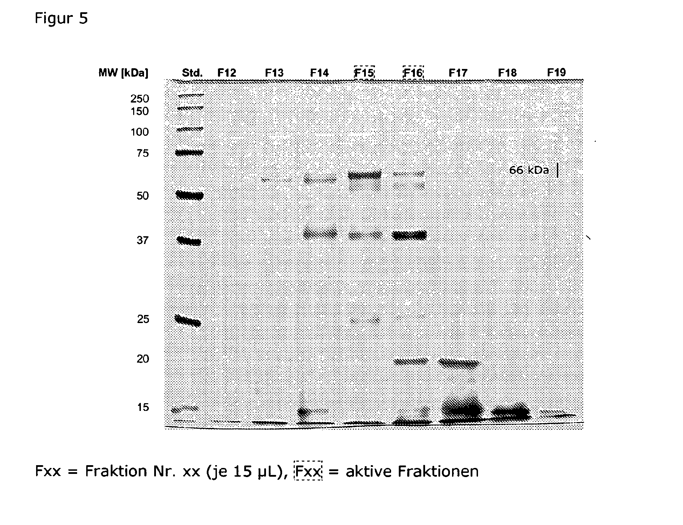

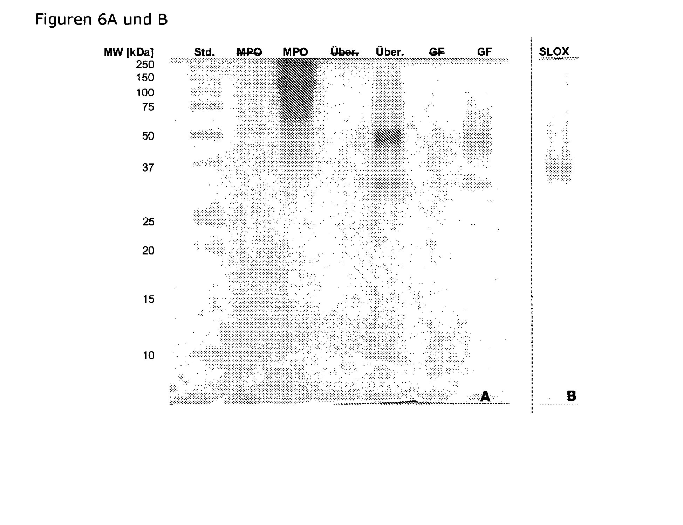

Für die SDS-Analysen wurden die Fraktionen 12 bis 19 mit E-Pure-Wasser umgepuffert und auf 80 µL konzentriert (Figur 5). Diese Konzentrate wurden zur denaturierenden SDS-PAGE mit anschließender Silberfärbung, zur nicht-denaturierenden SDS-PAGE mit anschließender spezifischer Färbung für Häm-/Metallenzyme (Figur 6) und für die Sequenzierung ausgewählter Proteinbanden nach denaturierenden SDS-PAGE und anschließender Färbung mittels Coomassie eingesetzt.

**Tabelle 4: FPLC mit UV-Detektor**

| | |
|---|---|
| FPLC-System | Biologic Duo Flow Chromatography System (Bio-Rad) mit Biologic Fraktionssammler Modell 2128 (Bio-Rad) |
| Datenaufnahme | Biologic Duo Flow Workstation (Bio-Rad), Version 3.00 |
| Detektionswellenlänge | 280 nm |

### Ionenaustauschchromatographie (IEX)

**Tabelle 5: Enzymreinigung mittels DEAE, Stufengradient**

| Säule | HiPrep 16/10 DEAE FF (Amersham Pharmacia Biotech) |
|---|---|
| Säulenvolumen (CV) | 20 mL |
| Startpuffer (A) | 20 mM TRIS-HCl, pH 7,5 |
| Elutionspuffer (B) | 20 mM TRIS-HCl + 0,2 M NaCl, pH 7,5 |
| Spülpuffer (C) | 20 mM TRIS-HCl + 2,0 M NaCl, pH 7,5 |
| Probenschleife | 5,0 mL |
| Flussrate | 5,0 mL min⁻¹ |
| Elutionsprogramm | 2 CV 100% Puffer A |
| | Probeninjektion: 2 x Probenvolumen (Puffer A) |
| | 7 CV100% Puffer A |
| | 6 CV35% Puffer B |
| | 5 CV100% Puffer B |
| | 4 CV100% Puffer C |
| | 5 CV100% Puffer A |
| Fraktionsvolumen | 2,0 mL |

**Tabelle 6: Enzymreinigung mittels SP FF**

| Säule | HiTrap SP Sepharose FF (Amersham Pharmacia Biotech) |
|---|---|
| Säulenvolumen (CV) | 1 mL |
| Startpuffer (A) | 20 mM Natriumcitrat, pH 3,0 |
| Elutionspuffer (B) | 20 mM Natriumcitrat + 1,0 M NaCl, pH 3,0 |
| Probenschleife | 2,0 mL oder 5,0 mL |
| Flussrate | 1,0 mL min⁻¹ |
| Elutionsprogramm | 5 CV 100% Puffer A |
| | Probeninjektion: 2 x Probenvolumen (Puffer A) |
| | 10 CV 100% Puffer A |
| | Linearer Gradient von 100% Puffer A (0% Puffer B) auf 0% Puffer A (100% Puffer B) über 15 CV |
| | 5 CV100% Puffer B |
| | 10 CV100% Puffer A |
| Fraktionsvolumen | 2,0 mL |

**Tabelle 7: Enzymreiniqunq mittels Superdex 200**

| Säule | Superdex 200 10/300 GL (Amersham Pharmacia Biotech) |
|---|---|
| Bettvolumen | ca. 24 mL |
| Trennbereich | 10 - 600 kDa |
| Probenschleife | 200 µL |
| Flussrate | 0,5 mL min⁻¹ |
| Laufpuffer | 200 mM TRIS-HCl, pH 7,5 |
| Elutionsprogramm | Probeninjektion: 2 x Probenvolumen (Laufpuffer) |
| | 30 mL 100% Laufpuffer |
| Fraktionsgröße | 1,0 mL |

Die Kalibration der Gelfiltrationssäule wurde mit Hilfe von Standardproteinen zweier Gelfiltration-Kalibrationskits (High Molecular Weight und Low Molecular Weight, Amersham Pharmacia Biotech) nach Herstellerangaben durchgeführt.

### b) SDS-PAGE unter denaturierenden Bedingungen

Die Proteine wurden in 12%igen Trenngelen getrennt (modifiziert nach Laemmmli, 1970) und mittels Coomassiefärbung oder Silberfärbung (Blum et al., 1987) visualisiert. Beide Methoden sind fachkundigen Personen bekannt.

### c) SDS-PAGE unter nicht-denaturierenden Bedingungen

Die SDS-PAGE unter nicht denaturierenden Bedingungen erfolgte analog Punkt 4b, jedoch wurde die Zusammensetzung des Auftragspuffers (Tabelle 8) modifziert.

**Tabelle 8: Zusammensetzung des Auftragpuffers**

| | |
|---|---|
| TRIS-HCl, pH 6,8 | 0,1 M |
| SDS | 20 g L⁻¹ |
| Bromphenolblau | 2 g L⁻¹ |
| Glycerol | 200 g L⁻¹ |
| Der Puffer wurde in Aliquots bei -20°C gelagert | |

### d) Färbung für Häm- und Metallenzyme (Hämfärbung)

Die Hämfärbung (modifiziert nach Thomas *et al.* 1976 und Henne *et al.* 2001) wurde nur nach SDS-PAGE unter nicht-denaturierenden Bedingungen durchgeführt. Für die Hämfärbung wurden folgende Lösungen verwendet (Tabelle 9):

**Tabelle 9: Zusammensetzung der Lösungen für die Hämfärbung**

| | |
|---|---|
| Lösung I | 6,3 mM methanolische TMBZ-Lösung |
| Lösung II | 0,25 M Natriumacetat-Puffer, pH 5,0 |
| Lösung III (Färbelösung) | Lösung I/Lösung II (3/7 v/v) |
| Lösung IV (Lagerlösung) | Lösung II/Isopropanol (7/3 v/v) |

Lösung I und III wurden jeweils kurz vor der Verwendung hergestellt.

SDS-Gele wurden für 45 min - 1 h in Lösung III im Dunkeln inkubiert, anschließend H₂O₂ (30%ig) bis zur Endkonzentration von 30 mM zugeben und für 1 min inkubiert. Die Gele wurden dreimal je 20 s in E-Pure-Wasser gewaschen. Verglichen wurde separiertes Lyophilisat von *Pleurotus sapidus* mit der über drei chromatographische Stufen gereinigten Enzymprobe (siehe Beispiel 3, Reinigung über drei chromatographische Stufen (IEX, IEX, GF)). Als Positivkontrollen dienten das hämhaltige Enzym Meerrettich-Peroxidase und eine Lipoxygenase aus Sojabohnen (Figur 6B). Anhand der spezifischen Färbung wurden zwei Proteine als Metallenzyme identifiziert, die ebenfalls nach der chromatographischen Reinigung über drei Stufen nachgewiesen werden konnten. Ein Erhitzen der Proben vor der Applikation führte jeweils zum Verlust der Aktivität.

### Beispiel 5: Peptidsequenzen

Analog zu Beispiel 4a wurde eine dreistufige chromatographische Reinigung des separierten Überstandes von *Pleurotus sapidus* durchgeführt. Nach der Proteinreinigung wurde durch Inkubation von (+)-Valencen mittels Gaschromatographie überprüft, ob die gereinigten Fraktionen Aktivität aufwiesen. Die aktiven Fraktionen 15 und 16 der abschließenden Gelfiltration wurden auf einem SDS-Gel unter denaturierenden Bedingungen getrennt und mit Coomassie^{®} R gefärbt (vergleiche analoge SDS-PAGE mit Silberfärbung in Figur 5). Die für die gesuchte Transformationsaktivität verantwortliche Bande, mit berechnetem Molekulargewicht von 66 kDa, wurde aus dem Gel ausgeschnitten und nach Trypsinverdau einer *de novo* Sequenzierung mittels "Electro-spray-tandem-Massenspektrometrie" (ESI-MS-MS) zugeführt (Tabelle 10).

**Tabelle 10: Ermittelte Peptidsequenzen (Einbuchstabencode) nach ESI-MS-MS-Analyse der über 3 Stufen gereinigten Enzyme; MW = Molekulargewicht nach SDS-PAGE, fettgedruckt = sichere Identifizierung**

| **MW [kDa]** | 66 | |
|---|---|---|
| **Peptidsequenzen** | **GFPVDQLNSPK** | (66-1) |
| | **DLNDMWTTLGAK** | (66-2) |
| | **YTESDLMAALPLNAK** | (66-3) |
| | **TNPLDLSVNQANDWPWR** | (66-4) |

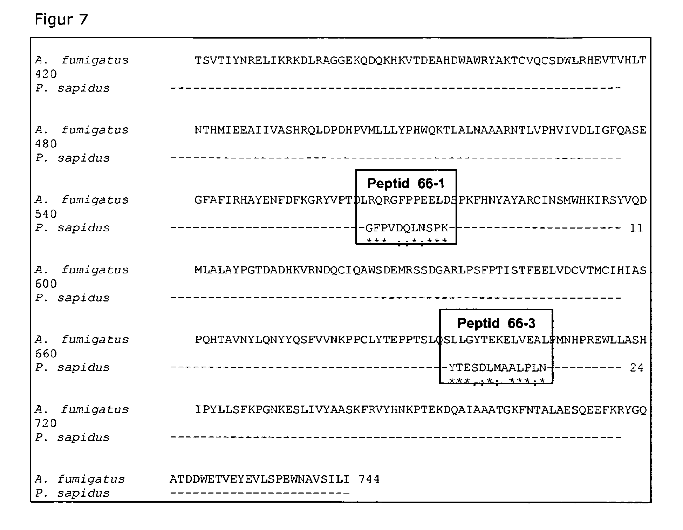

Homologievergleiche wurden durch Datenbankrecherche mit NCBI Blast mittels *blastp* (Schäffer *et al.* 2001) durchgeführt und Treffer auf verschiedene Lipoxygenasen aus Ascomyceten gefunden. Ausgehend von einem Vergleich der Peptidsequenzen 66-1 und 66-3 mit der Sequenz einer Lipoxygenase aus *Aspergillus fumigatus* (XP_746844.1; Figur 7) wurden degenerierte Primer abgeleitet und im PCR-Screening eingesetzt (Beispiel 6, Molekulare Charakterisierung, cDNA-Synthese und PCR-Screening).

### Beispiel 6: Molekulare Charakterisierung

### a) cDNA-Synthese und PCR-Screening

Für die Klonierung der Oxygenase-kodierenden cDNA-Sequenzen wurde cDNA aus *P. sapidus* synthetisiert und mittels Polymerase-Kettenreaktion gescreent. Das Myzel von *P. sapidus* wurde am 4. Kulturtag geerntet. Zur Isolierung der Gesamt-RNA wurde das RNeasy Plant Mini Kit (Qiagen, Hilden, Deutschland) nach Herstellerangaben verwendet. Die Qualität der isolierten RNA wurde mittels denaturierender Formaldehyd-Agarose-Gel-Elektrophorese und Färbung mit Ethidiumbromid überprüft. Für die Synthese der cDNA wurde der SMART^{™} PCR cDNA Synthesis Kit (Clontech-Takara Bio Europe, Saint-Germain-en-Laye, Frankreich) nach Herstellerangaben eingesetzt. Der Erststrang wurde mittels SuperScript™ II RNase H⁻ (Invitrogen, Karlsruhe, Deutschland) synthetisiert. PCR Primer wurden von Eurofins MWG Operon (Ebersberg, Deutschland) produziert. Für die PCR wurden ca. 20 ng cDNA als Template in 50 µL-Ansätzen mit 1x CoralLoad Puffer (Qiagen), 0,2 mM dNTPs, 0,4 µM Primer und 1,25 U HotStarTaq DNA Polymerase (Qiagen) verwendet. Die Amplifizierung wurde mit einem PCR Mastercycler personal (Eppendorf, Hamburg, Deutschland) durchgeführt. Die folgenden Primer wurden für die Gesamtsequenz der cDNA verwendet: forward (5'>AAA CCT GAT GAG GAG CTG TTT<3'); reverse (5'>ACA GGA TAC GGT GAT GAA TG<3').

### b) Klonierung und Sequenzierung von PCR-Produkten

Die PCR-Produkte wurden mit Hilfe des TA Cloning Kits (Invitrogen) in den Vektor pCR^{®}2.1 kloniert. Die Sequenzierung erfolgte mit M13 reverse und forward Primern und wurde von MWG Operon durchgeführt. Die erwartete Fragmentgröße im PCR-Screening bei Verwendung der degenerierten Primer gegeneinander betrug ca. 429 bp (entspricht 143 Aminosäuren). Die entsprechende Bande wurde aus dem Agarosegel isoliert, in den Vektor pCR2.1-TOPO zwischenkloniert und anschließend sequenziert. Die erhaltene Sequenz wurde erneut Homologievergleichen unterzogen und als Fragment einer Oxygenase identifiziert.

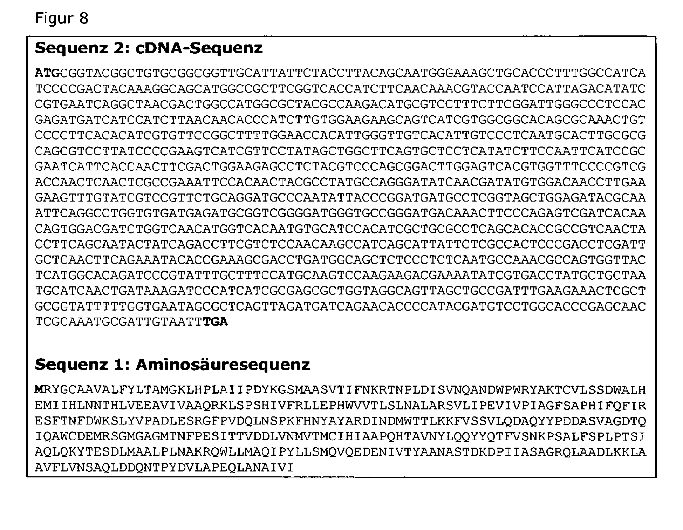

Durch *Primer Walking* wurde die kodierende Sequenz der cDNA mit einer Länge von 1191 bp beziehungsweise 396 Aminosäuren ermittelt (Figur 8). Die übersetzte Proteinsequenz zeigte eine Homologie von ∼ 50% zu einer Oxygenase aus dem Basidiomyceten *Laccaria bicolor* (ermittelt mit *blastp,* Schäffer *et al.* 2001).

### Literaturverzeichnis

Blum, H.; Beier, H.; Gross, H. J. (1987) Improved silver staining of plant proteins, RNA and DNA in polyacrylamide gels, Electrophoresis 8, 93-99.
Bouwmeester, H. J.; Kraker, J.-W. de; Schurink, M.; Bino, R. J.; Groot, A. de; Franssen, M. C. R. (2007) Plant Enzymes for Bioconversion, *Patent* US 7,214,507 B2.
Dhavlikar, R. S.; Albroscheit, G. (1973) Mikrobiologische Umsetzung von Terpenen: Valencen, Dragoco Rep 12, 251-258.
Drawert, F.; Berger, R. G.; Godelmann, R. (1984) Regioselective biotransformation of valencene in cell suspension cultures of Citrus sp., Plant Cell Rep 3, 37-40.
Furusawa, M.; Hashimoto, T.; Noma, Y.; Asakawa, Y. (2005) Highly efficient production of nootkatone, the grapefruit aroma from valencene, by biotransformation, Chem Pharm Bull (Tokyo) 53, 1513-1514.
Hashimoto, T.; Asakawa, Y.; Noma, Y.; Murakimi, C.; Tanaka, M.; Kanisawa, T.; Emura, M. (2003a) *Patent* JP 2001-267548 20010904.
Hashimoto, T.; Asakawa, Y.; Noma, Y.; Murakimi, C.; Tanaka, M.; Kanisawa, T.; Emura, M. (2003b) *Patent* JP 2002-51668 20020227.
Henne, K. R.; Kunze, K. L.; Zheng, Y.-M.; Christmas, P.; Soberman, R. J.; Rettie, A. E. (2001) Covalent Linkage of Prosthetic Heme to CYP4 Family P450 Enzymes, Biochemistry 40, 12925-12931.
Hitchman, T.; Robertson, D. E.; Hiraiwa, M.; Phillips, Y.; Gray, K. A. (2005) Laccases, nucleic acids encoding them and methods for making and using them, *Patent* WO/2005/021714.
Huang, R.; Christenson, P. A.; Labuda, I. M. (2001) Process for the preparation of nootkatone by laccase catalysis, *Patent* US 6200786.
Kaspera, R.; Krings, U.; Nanzad, T.; Berger, R. G. (2005) Bioconversion of (+)-valencene in submerged cultures of the ascomycete Chaetomium globosum, Appl Microbiol Biotechnol 67, 477-483.
Kraker, J.-W. de; Schurink, M.; Franssen, M. C. R.; König, W. A.; Groot, A. de; Bouwmeester, H. J. (2003) Hydroxylation of sesquiterpenes by enzymes from chicory (Cichorium intybus L.) roots, Tetrahedron 59, 409-418.
Muller, B.; Dean, C.; Schmidt, C.; Kuhn, J.-C. (1998) Process for the Preparation of Nootkatone, *Patent* US 5847226.
Müller, M.; Dirlam, K.; Henning, H.; Berger, R. G.; Krings, U.; Kaspera, R. (2005) Verfahren zur Herstellung von aromaaktiven Terpenen, *Patent* DE 10 2004 006 825 A1.
Ohloff, G. (1994) Scent and fragrances: The fascination of odors and their chemical perspectives. Berlin, Springer.
Okuda, M.; Sonohara, H.; Takigawa, H.; Tajima, K.; Ito, S. (1994) Nootkatone manufacture with *Rhodococcus* from valencen, *Patent* JP 06303967A.
Rio, J. A. del; Ortuño, A.; Puig, D. G.; Iborra, J. L.; Sabater, F. (1991) Accumulation of the sesquiterpenes nootkatone and valencene by callus cultures of Citrus paradisi, Citrus limonia and Citrus aurantium, Plant Cell Rep 10, 410-413.
Sakamaki, H.; Itoh, K.-i.; Taniai, T.; Kitanaka, S.; Takagi, Y.; Chai, W.; Horiuchi, C. A. (2005) Biotransformation of valencene by cultured cells of Gynostemma pentaphyllum, J Mol Catal B-Enzym 32, 103-106.
Schäffer, A. A.; Aravind, L.; Madden, T. L.; Shavirin, S.; Spouge, J. L.; Wolf, Y. I.; Koonin, E. V.; Altschul, S. F. (2001) Improving the accuracy of PSI-BLAST protein database searches with composition-based statistics and other refinements, Nucleic Acids Res 29, 2994-3005.
Schrader, J.; Berger, R. G. (2001) Biotechnological production of terpenoid flavor and fragrance compounds, In: Rehm, H.-J.; Reed, G. (Hg.) Biotechnology. Special processes, 10. Weinheim, Wiley-VCH, 374-422.
Sowden, R. J.; Yasmin, S.; Rees, N. H.; Bell, S. G.; Wong, L.-L. (2005) Biotransformation of the sesquiterpene (+)-valencene by cytochrome P450cam and P450BM-3, Org Biomol Chem 3, 57-64.
Sprecher, E. (1959) Über die Guttation bei Pilzen, Planta 53, 565-574.
Taubert, J.; Krings, U.; Berger, R. G. (2000) A comparative study on the disintegration of filamentous fungi, Journal of Microbiological Methods 42, 225-232.
Thomas, P. E.; Ryan, D.; Levin, W. (1976) An improved staining procedure for the detection of the peroxidase activity of cytochrome P-450 on sodium dodecyl sulfate polyacrylamide gels, Anal Biochem 75, 168-176.
Thompson, J. D.; Higgins, D. G.; Gibson, T. J. (1994) CLUSTAL W: improving the sensitivity of progressive multiple sequence alignment through sequence weighting, position-specific gap penalties and weight matrix choice, Nucleic Acids Res 22, 4673-4680.

## Patentansprüche

1. Polypeptid enthaltend eine Aminosäuresequenz gemäß SEQ ID No. 1 oder eine Variante, bei der bis zu 10% der Aminosäuren durch Insertionen, Deletionen oder Substitution verändert sind, die Terpenkohlenwasserstoffe oxidieren kann.

2. Nukleinsäure codierend für ein Polypeptid gemäß Anspruch 1.

3. Nukleinsäure nach Anspruch 2 mit einer Sequenz gemäß SEQ ID No. 2.

4. Nukleinsäure, die mit der Nukleinsäure gemäß einem der Ansprüche 2 oder 3 unter stringenten Bedingungen hybridisiert und deren Expressions-Produkt Terpenkohlenwasserstoffe oxidieren kann.

5. Vektor enthaltend eine Nukleinsäure nach einem der Ansprüche 2 bis 3.

6. Transformierter, nicht-menschlicher Organismus enthaltend einen Vektor nach Anspruch 5.

7. Verfahren zur Oxidation von Terpenkohlenwasserstoffen umfassend den Schritt des
- Inkontaktbringens von Terpenkohlenwasserstoffen mit einem Polypeptid gemäß Anspruch 1.

8. Verfahren nach Anspruch 7, wobei der Terpenkohlenwasserstoff Valencen ist und das aromaaktive Terpen Nootkaton ist.

## Claims

1. Polypeptide containing an amino acid sequence according to SEQ ID No. 1 or a variant, in which up to 10 % of said amino acids are modified by insertion, deletion or substitution, suitable for the oxidation of terpene hydrocarbons.

2. Nucleic acid coding for a polypeptide according to Claim 1.

3. Nucleic acid according to Claim 2, comprising a sequence according to SEQ ID No. 2.

4. Nucleic acid, hybridising with a nucleic acid according to Claims 2 or 3 under stringent conditions, whose expression products are suitable for the oxidation of terpene hydrocarbons.

5. Vector comprising a nucleic acid according to one of Claims 2 or 3.

6. Transformed non-human organism containing avector according to Claim 5.

7. Process for oxidation of terpene hydrocarbons encompassing the step of bringing a terpene hydrocarbon into contact with a polypeptide of Claim 1.

8. Process according to Claim 7, wherein said terpene hydrocarbon is valencene and the aroma-active terpene is nootkatone.

## Revendications

1. Polypeptide contenant une séquence d'acides aminés selon la séquence SEQ ID NO 1 ou une variante dans lequel jusqu'à 10% des acides aminés sont modifiés par des insertions, des délétions ou des substitutions, qui peut oxyder des hydrocarbures terpéniques.

2. Acide nucléique codant pour un polypeptide selon la revendication 1.

3. Acide nucléique selon la revendication 2 présentant une séquence selon la séquence SEQ. ID NO 2.

4. Acide nucléique, qui hybride avec l'acide nucléique selon l'une quelconque des revendications 2 ou 3 dans des conditions stringentes et dont le produit d'expression peut oxyder des hydrocarbures terpéniques.

5. Vecteur contenant un acide nucléique selon l'une quelconque des revendications 2 à 3.

6. Organisme transformé, non humain contenant un vecteur selon la revendication 5.

7. Procédé pour l'oxydation d'hydrocarbures terpéniques comprenant l'étape consistant à
- mettre en contact des hydrocarbures terpéniques avec un polypeptide selon la revendication 1.

8. Procédé selon la revendication 7, l'hydrocarbure terpénique étant le valencène et le terpène aromatique étant la Nootkatone.


### SEQUENCE LISTING

<110> N-Zyme Biotec GmbH
<120> Enzymatische Synthese von Nootkaton
<130> 082008ep
<160> 10
<170> PatentIn version 3.3
<210> 1
   <211> 396
   <212> PRT
   <213> Pleurotus sapidus
<400> 1
   Met Arg Tyr Gly Cys Ala Ala Val Ala Leu Phe Tyr Leu Thr Ala Met
   1               5                   10                  15
   Gly Lys Leu His Pro Leu Ala Ile Ile Pro Asp Tyr Lys Gly Ser Met
               20                  25                  30
   Ala Ala Ser Val Thr Ile Phe Asn Lys Arg Thr Asn Pro Leu Asp Ile
           35                  40                  45
   Ser Val Asn Gln Ala Asn Asp Trp Pro Trp Arg Tyr Ala Lys Thr Cys
       50                  55                  60
   Val Leu Ser Ser Asp Trp Ala Leu His Glu Met Ile Ile His Leu Asn
   65                  70                  75                  80
   Asn Thr His Leu Val Glu Glu Ala Val Ile Val Ala Ala Gln Arg Lys
                   85                  90                  95
   Leu Ser Pro Ser His Ile Val Phe Arg Leu Leu Glu Pro His Trp Val
               100                 105                 110
   Val Thr Leu Ser Leu Asn Ala Leu Ala Arg Ser Val Leu Ile Pro Glu
           115                 120                 125
   Val Ile Val Pro Ile Ala Gly Phe Ser Ala Pro His Ile Phe Gln Phe
       130                 135                 140
   Ile Arg Glu Ser Phe Thr Asn Phe Asp Trp Lys Ser Leu Tyr Val Pro
   145                 150                 155                 160
   Ala Asp Leu Glu Ser Arg Gly Phe Pro Val Asp Gln Leu Asn Ser Pro
                   165                 170                 175
   Lys Phe His Asn Tyr Ala Tyr Ala Arg Asp Ile Asn Asp Met Trp Thr
               180                 185                 190
   Thr Leu Lys Lys Phe Val Ser Ser Val Leu Gln Asp Ala Gln Tyr Tyr
           195                 200                 205
   Pro Asp Asp Ala Ser Val Ala Gly Asp Thr Gln Ile Gln Ala Trp Cys
       210                 215                 220
   Asp Glu Met Arg Ser Gly Met Gly Ala Gly Met Thr Asn Phe Pro Glu
   225                 230                 235                 240
   Ser Ile Thr Thr Val Asp Asp Leu Val Asn Met Val Thr Met Cys Ile
                   245                 250                 255
   His Ile Ala Ala Pro Gln His Thr Ala Val Asn Tyr Leu Gln Gln Tyr
               260                 265                 270
   Tyr Gln Thr Phe Val Ser Asn Lys Pro Ser Ala Leu Phe Ser Pro Leu
           275                 280                 285
   Pro Thr Ser Ile Ala Gln Leu Gln Lys Tyr Thr Glu Ser Asp Leu Met
       290                 295                 300
   Ala Ala Leu Pro Leu Asn Ala Lys Arg Gln Trp Leu Leu Met Ala Gln
   305                 310                 315                 320
   Ile Pro Tyr Leu Leu Ser Met Gln Val Gln Glu Asp Glu Asn Ile Val
                   325                 330                 335
   Thr Tyr Ala Ala Asn Ala Ser Thr Asp Lys Asp Pro Ile Ile Ala Ser
               340                 345                 350
   Ala Gly Arg Gln Leu Ala Ala Asp Leu Lys Lys Leu Ala Ala Val Phe
           355                 360                 365
   Leu Val Asn Ser Ala Gln Leu Asp Asp Gln Asn Thr Pro Tyr Asp Val
       370                 375                 380
   Leu Ala Pro Glu Gln Leu Ala Asn Ala Ile Val Ile
   385                 390                 395
<210> 2
   <211> 1191
   <212> DNA
   <213> Pleurotus sapidus
<400> 2
   atgcggtacg gctgtgcggc ggttgcatta ttctacctta cagcaatggg aaagctgcac     60
   cctttggcca tcatccccga ctacaaaggc agcatggccg cttcggtcac catcttcaac    120
   aaacgtacca atccattaga catatccgtg aatcaggcta acgactggcc atggcgctac    180
   gccaagacat gcgtcctttc ttcggattgg gccctccacg agatgatcat ccatcttaac    240
   aacacccatc ttgtggaaga agcagtcatc gtggcggcac agcgcaaact gtccccttca    300
   cacatcgtgt tccggctttt ggaaccacat tgggttgtca cattgtccct caatgcactt    360
   gcgcgcagcg tccttatccc cgaagtcatc gttcctatag ctggcttcag tgctcctcat    420
   atcttccaat tcatccgcga atcattcacc aacttcgact ggaagagcct ctacgtccca    480
   gcggacttgg agtcacgtgg tttccccgtc gaccaactca actcgccgaa attccacaac    540
   tacgcctatg ccagggatat caacgatatg tggacaacct tgaagaagtt tgtatcgtcc    600
   gttctgcagg atgcccaata ttacccggat gatgcctcgg tagctggaga tacgcaaatt    660
   caggcctggt gtgatgagat gcggtcgggg atgggtgccg ggatgacaaa cttcccagag    720
   tcgatcacaa cagtggacga tctggtcaac atggtcacaa tgtgcatcca catcgctgcg    780
   cctcagcaca ccgccgtcaa ctaccttcag caatactatc agaccttcgt ctccaacaag    840
   ccatcagcat tattctcgcc actcccgacc tcgattgctc aacttcagaa atacaccgaa    900
   agcgacctga tggcagctct ccctctcaat gccaaacgcc agtggttact catggcacag    960
   atcccgtatt tgctttccat gcaagtccaa gaagacgaaa atatcgtgac ctatgctgct   1020
   aatgcatcaa ctgataaaga tcccatcatc gcgagcgctg gtaggcagtt agctgccgat   1080
   ttgaagaaac tcgctgcggt atttttggtg aatagcgctc agttagatga tcagaacacc   1140
   ccatacgatg tcctggcacc cgagcaactc gcaaatgcga ttgtaatttg a             1191
<210> 3
   <211> 11
   <212> PRT
   <213> Pleurotus sapidus
<400> 3
   Gly Phe Pro Val Asp Gln Leu Asn Ser Pro Lys
   1               5                   10
<210> 4
   <211> 12
   <212> PRT
   <213> Pleurotus sapidus
<400> 4
   Asp Leu Asn Asp Met Trp Thr Thr Leu Gly Ala Lys
   1               5                   10
<210> 5
   <211> 15
   <212> PRT
   <213> Pleurotus sapidus
<400> 5
   Tyr Thr Glu Ser Asp Leu Met Ala Ala Leu Pro Leu Asn Ala Lys
   1               5                   10                  15
<210> 6
   <211> 17
   <212> PRT
   <213> Pleurotus sapidus
<400> 6
   Thr Asn Pro Leu Asp Leu Ser Val Asn Gln Ala Asn Asp Trp Pro Trp
   1               5                   10                  15
   Arg
<210> 7
   <211> 21
   <212> DNA
   <213> Artificial
<220>
   <223> Primer
<400> 7
   aaacctgatg aggagctgtt t 21
<210> 8
   <211> 20
   <212> DNA
   <213> Artificial
<220>
   <223> primer
<400> 8
   acaggatacg gtgatgaatg 20
<210> 9
   <211> 384
   <212> PRT
   <213> Aspergillus fumigatus
<400> 9
   Thr Ser Val Thr Ile Tyr Asn Arg Glu Leu Ile Lys Arg Lys Asp Leu
   1               5                   10                  15
   Arg Ala Gly Gly Glu Lys Gln Asp Gln Lys His Lys Val Thr Asp Glu
               20                  25                  30
   Ala His Asp Trp Ala Trp Arg Tyr Ala Lys Thr Cys Val Gln Cys Ser
           35                  40                  45
   Asp Trp Leu Arg His Glu Val Thr Val His Leu Thr Asn Thr His Met
       50                  55                  60
   Ile Glu Glu Ala Ile Ile Val Ala Ser His Arg Gln Leu Asp Pro Asp
   65                  70                  75                  80
   His Pro Val Met Leu Leu Leu Tyr Pro His Trp Gln Lys Thr Leu Ala
                   85                  90                  95
   Leu Asn Ala Ala Ala Arg Asn Thr Leu Val Pro His Val Ile Val Asp
               100                 105                 110
   Leu Ile Gly Phe Gln Ala Ser Glu Gly Phe Ala Phe Ile Arg His Ala
           115                 120                 125
   Tyr Glu Asn Phe Asp Phe Lys Gly Arg Tyr Val Pro Thr Asp Leu Arg
       130                 135                 140
   Gln Arg Gly Phe Pro Pro Glu Glu Leu Asp Ser Pro Lys Phe His Asn
   145                 150                 155                 160
   Tyr Ala Tyr Ala Arg Cys Ile Asn Ser Met Trp His Lys Ile Arg Ser
                   165                 170                 175
   Tyr Val Gln Asp Met Leu Ala Leu Ala Tyr Pro Gly Thr Asp Ala Asp
               180                 185                 190
   His Lys Val Arg Asn Asp Gln Cys Ile Gln Ala Trp Ser Asp Glu Met
           195                 200                 205
   Arg Ser Ser Asp Gly Ala Arg Leu Pro Ser Phe Pro Thr Ile Ser Thr
       210                 215                 220
   Phe Glu Glu Leu Val Asp Cys Val Thr Met Cys Ile His Ile Ala Ser
   225                 230                 235                 240
   Pro Gln His Thr Ala Val Asn Tyr Leu Gln Asn Tyr Tyr Gln Ser Phe
                   245                 250                 255
   Val Val Asn Lys Pro Pro Cys Leu Tyr Thr Glu Pro Pro Thr Ser Leu
               260                 265                 270
   Gln Ser Leu Leu Gly Tyr Thr Glu Lys Glu Leu Val Glu Ala Leu Pro
           275                 280                 285
   Met Asn His Pro Arg Glu Trp Leu Leu Ala Ser His Ile Pro Tyr Leu
       290                 295                 300
   Leu Ser Phe Lys Pro Gly Asn Lys Glu Ser Leu Ile Val Tyr Ala Ala
   305                 310                 315                 320
   Ser Lys Phe Arg Val Tyr His Asn Lys Pro Thr Glu Lys Asp Gln Ala
   325 330 335
   Ile Ala Ala Ala Thr Gly Lys Phe Asn Thr Ala Leu Ala Glu Ser Gln
   340 345 350
   Glu Glu Phe Lys Arg Tyr Gly Gln Ala Thr Asp Asp Trp Glu Thr Val
   355 360 365
   Glu Tyr Glu Val Leu Ser Pro Glu Trp Asn Ala Val Ser Ile Leu Ile
   370 375 380
<210> 10
   <211> 13
   <212> PRT
   <213> Pleurotus sapidus
<400> 10
   Tyr Thr Glu Ser Asp Leu Met Ala Ala Leu Pro Leu Asn
   1 5 10